**Europäisches Patentamt**

**European Patent Office**

**Office européen des brevets**

(19)

(11) Veröffentlichungsnummer: **0 052 108**
**B1**

(12) **EUROPÄISCHE PATENTSCHRIFT**

(45) Veröffentlichungstag der Patentschrift:
**06.11.85**

(51) Int. Cl.⁴: **A 61 F 5/44**

(21) Anmeldenummer: **81900986.1**

(22) Anmeldetag: **30.04.81**

(86) Internationale Anmeldenummer:
**PCT/CH 81/00046**

(87) Internationale Veröffentlichungsnummer:
**WO 81/03273 (26.11.81 Gazette 81/28)**

(54) **URIN-AUFNAHMEBEUTEL FÜR MÄNNLICHE, INKONTINENTE PERSONEN.**

(30) Priorität: **22.05.80 CH 3993/80**

(43) Veröffentlichungstag der Anmeldung:
**26.05.82 Patentblatt 82/21**

(45) Bekanntmachung des Hinweises auf die Patenterteilung:
**06.11.85 Patentblatt 85/45**

(84) Benannte Vertragsstaaten:
**AT DE FR GB NL SE**

(56) Entgegenhaltungen:
**EP - A - 0 032 138**
**DE - C - 327 495**
**FR - A - 1 232 413**
**FR - A - 1 445 658**
**US - A - 2 310 505**
**US - A - 3 526 227**

(73) Patentinhaber: **NUSSBAUMER, Max,**
**Scheibenstrasse 13, CH-3000 Bern 22 (CH)**

(72) Erfinder: **NUSSBAUMER, Max, Scheibenstrasse 13,**
**CH-3000 Bern 22 (CH)**

(74) Vertreter: **Gasser, François W.,**
**Hirschengraben 10 Postfach 1555, CH-3001 Bern (CH)**

ACTORUM AG

## Beschreibung

Die vorliegende Erfindung betrifft eine Urin-Aufnahme-Vorrichtung für inkontinente männliche Personen, bestehend aus einem Urin-Aufnahme-Beutel, an dessen oberem, offenem Ende ein Haltering befestigt ist, der eine äussere Ringnut zur Aufnahme eines Verbindungsstückes einer Tragbandage aufweist und dessen unteres Ende in einem konischen Trichter ausläuft, in welchen von innen ein Auslaufstutzen eingeschoben ist.

Es sind Urin-Aufnahme-Vorrichtungen bekannt, welche insbesondere in bezug auf die Bereitstellung erhebliche Nachteile aufweisen. So verursacht beispielsweise das Stülpen des Beutels über Halteringe grosse Schwierigkeiten. Unangenehm für den Benützer wirkt sich auch das Undichtwerden der Beutel aus, indem die Verschweissung des Auslaufstutzens mit dem Beutel diesbezüglich einen ausgesprochenen Schwachpunkt darstellt.

Im wesentlichen kennt man aus der US-PS 3 526 227 einen Urin-Beutel für inkontinente männliche Personen, bei dem ein Urin-Aufnahme-Beutel an dessen oberem, offenem Ende mit einem Haltering verbunden ist und dessen unteres Ende in einen Trichter ausläuft. In letzteren ist von innen ein Auslaufstutzen eingeschoben.

Dieser bekannte Urin-Beutel weist mehrere Nachteile auf. So verfügt er über einen als Schraubventil ausgebildeten Auslass, der praktisch nur zweihändig bedienbar ist. Zudem ist das obere Ende des Beutels sehr aufwendig gestaltet, ohne allerdings mit Sicherheit das unerwünschte Ausfliessen von Urin, insbesondere bei liegenden Patienten, zu verhindern. Ferner ist das Einsetzen des Ventils in den Beutel nahezu undurchführbar.

Aus der DE-C 327 495 ist ferner eine stülpbare Abdichtungshülse bekannt, die eine Abdichtung des Beutels gegen aussen derart erreichen soll, dass sie sich um das männliche Glied schmiegt. Eine sich über den grössten Teil des Gliedes erstreckende elastische Hülse bringt Probleme mit sich, insbesondere wenn sie sich in einer Vorrichtung wie derjenigen gemäss dieser Schrift befindet, da sie als alleinige Dichtung ungenügend ist.

Der vorliegenden Erfindung liegt daher die Aufgabe zugrunde, eine Urin-Aufnahme-Vorrichtung der eingangs beschriebenen Art zu schaffen, die keinen der Nachteile der bekannten Urin-Beutel aufweist und die in der Handhabung und im Tragen sehr einfach ist und grösstmögliche Sicherheit gegen unerwünschtes Auslaufen von Urin gewährleistet.

Erfindungsgemäss wird diese Aufgabe durch eine Urin-Aufnahme-Vorrichtung der eingangs beschriebenen Art gelöst, die dadurch gekennzeichnet ist, dass zur Bildung der äusseren Ringnut der Querschnitt des Halterings U-förmig ist, wobei das offene Ende der U-Form nach aussen gerichtet ist, der Haltering mit dem Beutel verschweisst ist und eine über ihn gestülpte Penis-Manschette trägt, und dass das Verbindungsstück der Tragbandage ein Kunststoffring ist, der zusammen mit dem einen Ende der Penis-Manschette in der Ringnut des Halterings liegt, und dass der Auslaufstutzen mittels eines elastischen Dichtungs- und Halteringes im trichterförmigen Auslauf des Beutels befestigt ist.

In der folgenden Beschreibung ist eine vorteilhafte Ausführungsvariante der erfindungsgemässen Vorrichtung anhand der Zeichnung beschrieben. In der Zeichnung zeigt:

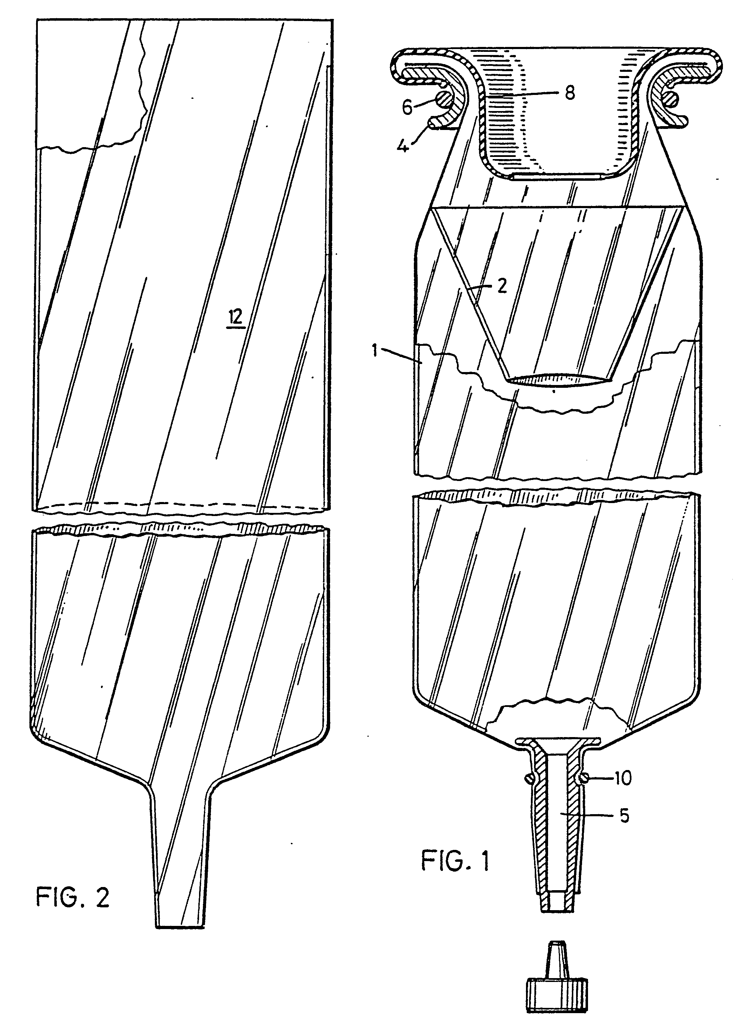

Fig. 1 eine Ansicht der Vorrichtung,
Fig. 2 einen darüber stülpbaren Sicherheits-Beutel,

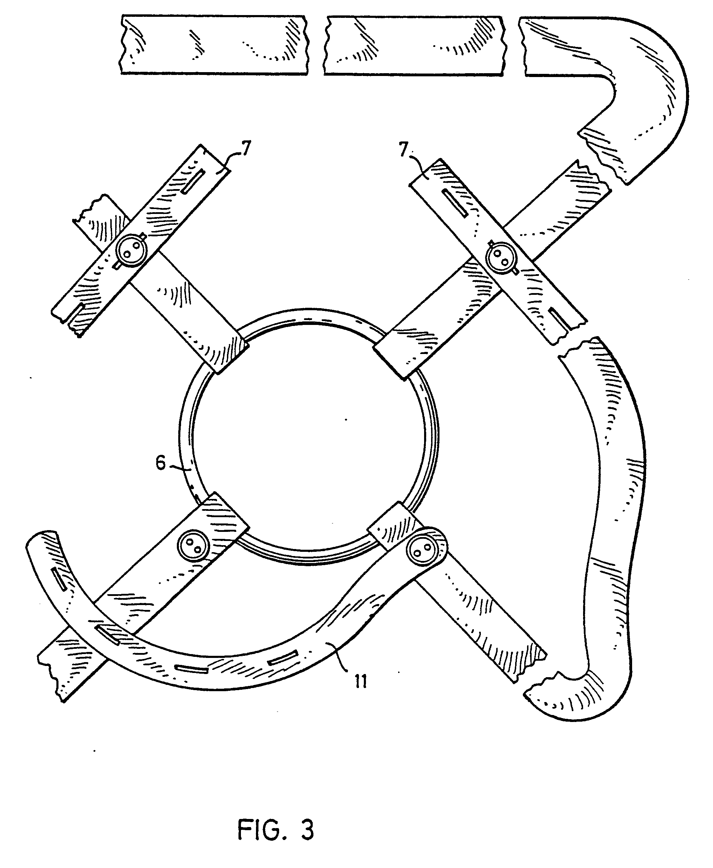

Fig. 3 eine Tragbandage.

Am oberen, offenen Ende eines vornehmlich aus Kunststoff gefertigten auswechselbaren Urin-Aufnahme-Beutels 1 mit Rücklaufventil 2 ist ein Haltering 4 aus Hartkunststoff angeschweisst. Der Haltering 4 dient ferner zur Aufnahme eines Verbindungsstückes 6 einer Tragbandage 7, einer Penis-Manschette 8 und eines Sicherheits-Beutels 12. Das Verbindungsstück 6 der Tragbandage 7 und das obere Ende des Sicherheits-Beutels 12 lagern in einer äusseren, im Querschnitt U-förmigen Ringnut des Halterings 4. Die Penis-Manschette 8 wird vor Gebrauch von oben über den Haltering 4 gestülpt. Der Urin-Aufnahme-Beutel 1 läuft nach unten trichterförmig aus. Um das Einschweissen eines Hartstutzens zu umgehen, wird ein Auslaufstutzen 5, der in einer äusseren Ringnut einen elastischen Dichtungs- und Haltering 10 trägt, ohne Verschweissung von innen in den Urin-Aufnahme-Beutel 1 eingeschoben.

Ein einen Teil der Tragbandage 7 bildendes Hodenband 11 wird beim Anziehen der erfindungsgemässen Vorrichtung hinter den Hoden durchgeführt. Es verhindert das Ausschlüpfen des Penis bei gewissen Körperbewegungen aus der Vorrichtung. Ohne Veränderung des Urin-Aufnahme-Beutels 1 kann ein Sicherheits-Beutel 12 zwecks Erreichung einer absoluten Sicherheit bezüglich Abdichtung über den Urin-Aufnahme-Beutel 1 übergezogen und am Haltering 4 befestigt werden.

Die erfindungsgemässe Urin-Aufnahme-Vorrichtung weist gegenüber den bekannten Urin-Sammlern den Vorteil auf, dass ein Überstülpen des Urin-Aufnahme-Beutels 1 über einen Haltering überflüssig wird, was ihre Handlichkeit wesentlich erhöht. Zudem bietet sie grösstmögliche Sicherheit gegen das ungewollte Auslaufen von Urin.

## Patentansprüche

1. Urin-Aufnahme-Vorrichtung für inkontinente männliche Personen, bestehend aus einem Urin-Aufnahme-Beutel (1), an dessen oberem, offenem Ende ein Haltering (4) befestigt ist, der eine äussere Ringnut zur Aufnahme eines Verbindungsstückes (6) einer Tragbandage (7) aufweist und dessen unteres Ende in einem konischen Trichter ausläuft, in welchen von innen ein Auslaufstutzen (5) eingeschoben ist, dadurch gekennzeichnet, dass zur Bildung der äusseren Ringnut der Querschnitt des Halterings (4) U-förmig ist, wobei das offene Ende der U-Form nach aussen gerichtet ist, und dass der Haltering mit dem Beutel (1) verschweisst ist und eine über ihn gestülpte Penis-Manschette (8) trägt und dass das Verbindungsstück (6) der Tragbandage (7) ein Kunststoffring ist, der zusammen mit dem einen Ende der Penis-Manschette (8) in der Ringnut des Halterings (4) liegt, und dass der Auslaufstutzen (5) mittels eines elastischen Dichtungs- und Halteringes (10) im trichterförmigen Auslauf des Beutels (1) befestigt ist.

2. Urin-Aufnahme-Vorrichtung nach Anspruch 1, dadurch gekennzeichnet, dass die Tragbandage (7) ein Querband (11) aufweist, das hinter den Hoden des Patienten durchgezogen wird.

3. Urin-Aufnahme-Vorrichtung nach Anspruch 1, dadurch gekennzeichnet, dass über den Urin-Aufnahme-Beutel (1) ein Sicherheits-Beutel (12) anbringbar ist.

## Claims

1. Device for the reception of the urine of incontinent male persons, consisting of a pocket (1) for the reception of urine, fixed at the upper open end of said pocket there is a fastening ring (4) showing an outer annular groove for the reception of a connection piece (6) of a fastening bandage (7), and the lower end of said pocket tapers into a conical funnel, in which a discharge tube (5) is inserted from the interior, characterized in that the cross-section of the fastening ring (4) is U-shaped for the formation of an outer annular groove, the open end of the U-shape being directed towards the exterior and that the fastening ring is welded to the pocket (1) and bears a penis cuff (8) slipped over it, and that the connection piece (6) of the fastening bandage (7) is a ring made of synthetic material, which lies together with one end of the penis cuff (8) in the annular groove of the fastening ring (4), and that the discharge tube (5) is fixed in the funnel-shaped discharge of the pocket (1) by means of an elastic sealing and fastening ring (10).

2. Device for the reception of urine according to Claim 1, characterized in that the fastening bandage (7) has a transverse strap (11) which is passed through behind the testicles of the patient.

3. Device for the reception of urine according to Claim 1, characterized in that a safety pocket (12) is attachable to the pocket (1) for the reception of urine.

## Revendications

1. Dispositif pour recueillir l'urine d'un sujet incontinent de sexe masculin, comportant pour recevoir l'urine une poche (1) ayant une extrémité supérieure ouverte assujettie à une bague de fixation (4) qui présente une gorge annulaire extérieure, pour recevoir une pièce de liaison (6) d'un bandage porteur (7); l'extrémité de la poche à urine (1) se prolongeant par un appendice conique, dans lequel un élément tubulaire de sortie (5) est enfilé par l'intérieur; ce dispositif étant caractérisé en ce que la bande de fixation (4) présente, en section transversale, le profil d'un U, ayant son extrémité ouverte orientée vers l'extérieur, pour constituer une gorge annulaire extérieure; cette bague de fixation (4) étant assujettie par soudage à la poche à urine (1); cette bague (4) portant un manchon (8) adapté à recevoir la verge de l'utilisateur, et rabattu sur la bague; la pièce de liaison (6) du bandage porteur (7) étant constituée par un anneau en matière plastique, qui est engagé dans la gorge annulaire de la bague de fixation (4) avec l'une des extrémités du manchon (8) associé à la verge de l'utilisateur; et l'élément tubulaire de sortie (5) étant retenu dans l'appendice conique de la poche à urine (1) au moyen d'un anneau élastique d'étanchéité et de fixation (10).

2. Dispositif selon la revendication 1, caractérisé en ce que le bandage porteur (7) comporte une bande latérale (11) que l'on fait passer derrière le scroton de l'utilisateur.

3. Dispositif selon la revendication 1, caractérisé en ce qu'il comporte un sac auxiliaire de sécurité (12) prévu pour être disposé sur la poche à urine (1).

FIG. 2

FIG. 1

FIG. 3